# EUROPEAN PATENT SPECIFICATION

(11) **EP 2 252 334 B1**
(45) Date of publication and mention of the grant of the patent: **03.07.2013**
(21) Application number: 09717355.3
(22) Date of filing: 03.03.2009
(51) Int. Cl.: A61L 15/00, A61L 15/60, C08J 3/20, B01J 19/20

(54) **PROCESS FOR PRODUCING SUPERABSORBENTS**
VERFAHREN ZUR HERSTELLUNG VON SUPERABSORBERN
PROCÉDÉ POUR PRODUIRE DES SUPERABSORBANTS

(30) Priority: 05.03.2008 US 33834 P
(43) Date of publication of application: 24.11.2010
(73) Proprietor: BASF SE, 67056 Ludwigshafen (DE)
(72) Inventor: Hillebrecht, Annemarie, 36093 Künzell (DE); Bennett, Andrea Karen, 68161 Mannheim (DE); Peterson, Monte, Pearland TX 77584 (US); Herfert, Norbert, 63674 Altenstadt (DE); Dücker, Carolin Nadine, 67063 Ludwigshafen (DE); Chiang, William G-J, Charlotte NC 28226 (US); Woodrum, Tom, Midlothian Virginia 23114 (US); Young, Michael, Charlotte NC 28273 (US)
(86) International application number: PCT/EP2009/052483
(87) International publication number: WO 2009/109563

(56) References cited:
- WO-A-2004/022608
- WO-A-2007/074167
- US-A1- 2004 110 897

## Description

The present invention relates to a process for producing superabsorbents that exhibit superior permeability properties. In particular, the present invention relates to a kneader process for producing superabsorbents containing inorganic solids.

Superabsorbents are known. Superabsorbents are materials that are able to take up and retain several times their weight in water, possibly up to several hundred times their weight, even under moderate pressure. Absorbing capacity is usually lower for salt-containing solutions compared to distilled or otherwise de-ionised water. Typically, a superabsorbent has a centrifugal retention capacity ("CRC", method of measurement see hereinbelow) of at least 5 g/g, preferably at least 10 g/g and more preferably at least 15 g/g. Such materials are also commonly known by designations such as "high-swellability polymer", "hydrogel" (often even used for the dry form), "hydrogel-forming polymer", "water-absorbing polymer", "absorbent gel-forming material", "swellable resin", "water-absorbing resin" or the like. The materials in question are crosslinked hydrophilic polymers, in particular polymers formed from (co)polymerized hydrophilic monomers, graft (co)polymers of one or more hydrophilic monomers on a suitable grafting base, crosslinked ethers of cellulose or starch, crosslinked carboxymethylcellulose, partially crosslinked polyalkylene oxide or natural products that are swellable in aqueous fluids, examples being guar derivatives, of which water-absorbing polymers based on partially neutralized acrylic acid are most widely used. Superabsorbents are usually produced, stored, transported and processed in the form of dry powders of polymer particles, "dry" usually meaning less than 5 wt.-% water content (also called "moisture content", method of measurement see hereinbelow). A superabsorbent transforms into a gel on taking up a liquid, specifically into a hydrogel when as usual taking up water. By far the most important field of use of superabsorbents is the absorbing of bodily fluids. Superabsorbents are used for example in hygiene articles such as diapers for infants, incontinence products for adults or feminine hygiene products. Examples of other fields of use are as water-retaining agents in market gardening, as water stores for protection against fire, for liquid absorption in food packaging or, in general, for absorbing moisture.

Processes for producing superabsorbents are also known. The acrylate-based superabsorbents which dominate the market are produced by radical polymerisation of acrylic acid in the presence of a crosslinking agent (the "intemal crosslinker"), usually in the presence of water, the acrylic acid being neutralized to some degree in a neutralisation step conducted prior to or after polymerisation, or optionally partly prior to and partly after polymerisation, usually by adding a alkali, most often an aqueous sodium hydroxide solution. This yields a polymer gel which is comminuted (depending on the type of reactor used, comminution may be conducted concurrently with polymerisation) and dried. Usually, the dried powder thus produced (the "base polymer") is surface crosslinked (also termed surface "post"crosslinked) by adding further organic or polyvalent metal (i.e. cationic) crosslinkers to generate a surface layer which is crosslinked to a higher degree than the particle bulk. Most often, aluminium sulphate is being used as polyvalent metal crosslinker. Applying polyvalent metal cations to superabsorbent particles is sometimes not regarded as surface crosslinking, but termed "surface complexing" or as another form of surface treatment, although it has the same effect of increasing the number of bonds between individual polymer strands at the particle surface and thus increases gel particle stiffness as organic surface crosslinkers have. Organic and polyvalent metal surface crosslinkers can be cumulatively applied, jointly or in any sequence.

Surface crosslinking leads to a higher crosslinking density close to the surface of each superabsorbent particle. This addresses the problem of "gel blocking", which means that, with earlier types of superabsorbents, a liquid insult will cause swelling of the outermost layer of particles of a bulk of superabsorbent particles into a practically continuous gel layer, which effectively blocks transport of further amounts of liquid (such as a second insult) to unused superabsorbent below the gel layer. While this is a desired effect in some applications of superabsorbents (for example sealing underwater cables), it leads to undesirable effects when occurring in personal hygiene products. Increasing the stiffness of individual gel particles by surface crosslinking leads to open channels between the individual gel particles within the gel layer and thus facilitates liquids transport through the gel layer. Although surface crosslinking decreases the CRC or other parameters describing the total absorption capacity of a superabsorbent sample, it may well increase the amount of liquid that can be absorbed by hygiene product containing a given amount of superabsorbent.

Frederic L. Buchholz und Andrew T. Graham (Hrsg.) in: "Modern Superabsorbent Polymer Technology", J. Wiley & Sons, New York, U.S.A. / Wiley-VCH, Weinheim, Germany, 1997, ISBN 0-471-19411-5, give a comprehensive overview over superabsorbents and processes for producing superabsorbents.

Superabsorbents containing inorganic solids as fillers or additives are also known. Quite often, clays are used as inorganic solid fillers. These additives influence a variety of parameters.

The Buchholz/Graham reference work cited above, mentions in its section on additives for improved handling of solution-polymerised superabsorbents that a combination of particulate silica with polyols or polyalkylene glycols applied to (co)polymers of poly(acrylamide) reduces the rate of moisture absorption in humid environments.

GB 2 082 614 A discloses a blend comprising superabsorbent powder and an extender material selected from uncrosslinked cellulose derivatives, starch, certain clays and minerals, or mixtures thereof. This composition exhibits better absorbency than the calculated sum of the absorbencies of its components. US 6 376 034 B1 discloses a similar synergistic blend of a non-crosslinked and therefore water-soluble gel-forming absorbing compound, an trivalent cation and a clay. US 4 500 670 teaches a blend of superabsorbent and inorganic powder that exhibits increased gel strength. In the suspension process for producing a high-expansion type, gas-permeable superabsorbent of US 4 735 987, superabsorbent particles are crosslinked in the presence of an inorganic filler such as hydrotalcite, montmorillonite, talc, pyrophyllite or kaolinite.
US 4 914 066 discloses bentonite pellets comprising 0.5 to 15 wt.-% superabsorbent.

According to WO 91/12 029 A1, WO 91/12 031 A1 or EP 799 861 A1, water-insoluble zeolithes or activated carbon are used as additives to superabsorbents to avoid malodours. According to WO 01/13 965 A1, high-silicon zeolithes are used for this purpose.

US 5 419 956 discloses absorbent structures containing superabsorbent that is produced by solution polymerisation and, to improve liquids distribution, admixed with inorganic powders such as silica, alumina, titania or clays, for example kaolin and montmorillonite. WO 01/68 156 A1 describes adding alumosilicates, in particular lamellar alumosilicates such as saponite or montmorillonite or three-dimensional lattice alumosilicates such as certain zeolithes to superabsorbents prior to, during or after polymerisation, to improve liquids distribution and to avoid malodours.

In the process of US 3 900 378 for producing a superabsorbent by crosslinking superabsorbent particles using ionising radiation, a filler is employed to dispergate the polymer particles. Examples of suitable fillers include minerals such as perlite, diatomaceous earth, clay, fly ash and magnesium silicates. According to US 5 733 576 clay may be added to a superabsorbing blend of crosslinked polyacrylate particles and polysaccharide particles. One type of suitable reactor for producing this blend is a kneader.

US 6 124 391 discloses using inorganic powders, in particular clays such as kaolin, as anticaking agent for superabsorbents.

WO 00/72 958 A1 describes using clays as synergistic fillers in superabsorbents. Clay is added to the monomer mixture prior to polymerisation. WO 01/32 117 A1 teaches to use hydrotalcite as basic filler in a comparatively acidic superabsorbent to increase its tolerance against sodium chloride.

WO 2004/018 005 relates to a process for processing superabsorbents that comprises adding a clay to polymerized hydrogel particles, then neutralising and drying the gel to form dry superabsorbent. According to WO 2004/018 006, clay is added to superabsorbent particles during the surface crosslinking step. WO 2006/134 085 discloses a process for producing superabsorbents that comprises adding a particulate hydrophobic compound to the monomer solution, to the as-polymerised un-dried gel product or to both. Suitable hydrophobic compounds are polymethylmethacrylate, hydrophobic or hydrophobicised silica, alumina or clay minerals.

Kneaders are well known as polymerisation reactors for producing superabsorbents. Kneaders are machines designed to mix highly viscous media or produce highly viscous media from liquid, solid and/pr plastic components. Kneaders employ kneading tools that move with respect to each other or with respect to non-moving surfaces in a way that imparts high shearing forces to the kneader's content and repeatedly compresses, chops and laminarily dislocates it. Contrary to typical extruders and feeding/discharging screws that, in some designs, may have a certain mixing effect but primarily move their content along one predetermined direction, a typical kneader's primary effect is radially and longitudinally mixing its content, although some kneaders also may longitudinally move their contents. Kneaders therefore fall within the generic term "mixers" and sometimes are referred to as "kneading mixers" or, although tautologically, as "mixing kneaders". Kneaders may also be used for conducting reactions, typically reactions involving highly viscous media that need application of high shear forces, thus, the term "kneader reactor" is also common.

GB 2 146 343 A discloses a process employing two-arm or three-shaft kneaders for the polymerisation reaction. The kneader's kneading elements exert shear force, but do not move the product in a predetermined direction with respect to the kneader's axes or the kneading shafts, leading to a residence time characteristic of a stirred tank reactor with complete backmixing of components rather than that of a tubular reactor. Nevertheless, the kneader of GB 2 146 343 A may be operated continuously. In that case, product is discharged from the kneader by a separate discharge screw. US 5 164 459 discloses process for surface crosslinking a superabsorbent that includes adding a water-insoluble fine powder to the superabsorbent. The water-insoluble fine powder is selected from a range of inorganic or organic powders that includes silica, alumina, titania or clays. Both surface crosslinking and producing the base polymer may be conducted in a kneader.

US 4 769 427 relates to using a single shaft kneader for polymerising a monomer mixture to form a superabsorber hydrogel.

WO 03/022 896 A1 discloses a continuous polymerisation process for the manufacture of superabsorbents in a three-zone reactor.system comprising an initial backmixed zone where polymerisation of the monomer mixture is initalized, a downstream gelphase zone and a final gel granulate zone. The latter two zones contain at least two rotating shafts. All zones may be located in one reaction vessel such as a model ORP kneader available from List AG, Arisdorf, Switzerland.

WO 01/38 402 A1 discloses a process for producing superabsorbents in a two-shaft kneader that also comprises elements on the kneader's shafts that transport the kneader's content parallel to the shafts. The reaction heat is removed by a combination of water evaporation, product discharge and wall cooling. WO 2006/034 806 A1 relates to a similar process in which the kneader's filling level is at least 71 %, the hydroquinone semi-ether level in the monomer is below 150 ppm, the temperature in the kneader's polymerisation zone is at least 65 °C or the kneader's backmixing rate is less than 0.33. WO 2006/034 853 A1 discloses a kneader that also comprises elements on the kneader's shafts that transport the kneader's content parallel to the axes. This kneader is suitable for producing superabsorbents. WO 2007/003 619 A1 relates to a device for adding additives into a reaction vessel such as a kneader.

The superabsorbent fines that inevitably occur during superabsorbent production and are removed by sifting pose a general recycling problem. It has been known for a long time to recycle these fines into the polymerisation step. WO 2007/074 167 A2 discloses a process for producing a superabsorbent that comprises adding particulate additives such as clay and superabsorbent fines, in a manner where a first portion of additives is added into the reactor at a point corresponding to not more than 40 % of the total average residence time of the kneader's contents in the kneader, and a second portion is added at a point corresponding to at least 45 % of this time. The individual additive portions may be comprised of superabsorbent fines only or of a superabsorbent fines and clay mixture. Further, the first additive portion may consist of superabsorbent fines only while the second is a mixture.

It is an object of the present invention to provide an improved process for producing a superabsorbent that comprises adding an inorganic filler, in particular clay, and other additives, in particular superabsorbent fines, in a simple and effective manner.

We have found that this object is achieved by a process for producing superabsorbents by polymerisation in a kneader equipped with at least two parallel shafts that also comprises elements on at least one shaft that transport the kneader's content parallel to the shafts from a feed section to a discharge section, the process comprising the addition of an inorganic powder to the kneader's contents prior to or during polymerisation and the addition of at least one other additive to the kneader's contents during polymerisation, wherein the inorganic powder is added no later than half the average residence time of the kneader's contents and the other additive is added no earlier than half his residence time.

The superabsorbent in the present invention is a superabsorbent capable of absorbing and retaining amounts of water equivalent to many times its own weight under a certain pressure. In general, it has a centrifugal retention capacity (CRC, method of measurement see hereinbelow) of at least 5 g/g, preferably at least 10 g/g and more preferably at least 15 g/g. Preferably, the superabsorbent is a crosslinked polymer based on partially neutralized acrylic acid, and it is surface postcrosslinked. A "superabsorbent" can also be a mixture of chemically different individual superabsorbents in that it is not so much the chemical composition which matters as the superabsorbing properties.

Processes for producing superabsorbents, including surface-postcrosslinked superabsorbents, are known. The synthetic superabsorbents based on acid-functional monomers that presently dominate the market are typically obtained by polymerisation of a monomer solution comprising
a) at least one ethylenically unsaturated acid-functional monomer,
b) at least one crosslinker (usually designated the "internal" crosslinker(s)),
c) optionally one or more ethylenically and/or allylically unsaturated monomers copolymerizable with the monomer a), and
d) optionally one or more water-soluble polymers onto which the monomers a), b) and if appropriate c) can be at least partly grafted.

Suitable monomers a) are for example ethylenically unsaturated carboxylic acids, such as acrylic acid, methacrylic acid, maleic acid, fumaric acid and itaconic acid, or derivatives thereof, such as acrylamide, methacrylamide, acrylic esters and methacrylic esters. Acrylic acid and methacrylic acid are particularly preferred monomers. Acrylic acid is most preferable.

The monomers a) and especially acrylic acid comprise preferably up to 0.025% by weight of a hydroquinone half ether. Preferred hydroquinone half ethers are hydroquinone monomethyl ether (MEHQ) and/or tocopherols, in particular alpha-tocopherol, especially racemic alpha-tocopherol. Among the tocopherols, RRR-alpha-Tocopherol is particularly preferred.

The monomer solution comprises preferably not more than 130 weight ppm, more preferably not more than 70 weight ppm, preferably not less than 10 weight ppm, more preferably not less than 30 weight ppm and especially about 50 weight ppm of hydroquinone half ether, all based on acrylic acid, with acrylic acid salts being arithmetically counted as acrylic acid. For example, the monomer solution can be produced using an acrylic acid having an appropriate hydroquinone half ether content.

Crosslinkers b) are compounds having at least two polymerizable groups which can be free-radically interpolymerized into the polymer network. Useful crosslinkers b) include for example ethylene glycol dimethacrylate, diethylene glycol diacrylate, allyl methacrylate, trimethylolpropane triacrylate, triallylamine, tetraallyloxyethane as described in EP 530 438 A1, di- and triacrylates as described in EP 547 847 A1, EP 559 476 A1, EP 632 068 A1, WO 93/21 237 A1, WO 03/104 299 A1, WO 03/104 300 A1,
WO 03/104 301 A1 and DE 103 31 450 A1, mixed acrylates which, as well as acrylate groups, comprise further ethylenically unsaturated groups, as described in DE 103 31 456 A1 and WO 04/013 064 A2, or crosslinker mixtures as described for example in DE 195 43 368 A1, DE 196 46 484 A1, WO 90/15 830 A1 and WO 02/032 962 A2.

Useful crosslinkers b) include in particular N,N'-methylenebisacrylamide and N,N'-methylenebismethacrylamide, esters of unsaturated mono- or polycarboxylic acids of polyols, such as diacrylate or triacrylate, for example butanediol diacrylate, butanediol dimethacrylate, ethylene glycol diacrylate, ethylene glycol dimethacrylate and also trimethylolpropane triacrylate and allyl compounds, such as allyl (meth)acrylate, triallyl cyanurate, diallyl maleate, polyallyl esters, tetraallyloxyethane, triallylamine, tetraallylethylenediamine, allyl esters of phosphoric acid and also vinylphosphonic acid derivatives as described for example in EP 343 427 A2. Useful crosslinkers b) further include pentaerythritol diallyl ether, pentaerythritol triallyl ether, pentaerythritol tetraallyl ether, polyethylene glycol diallyl ether, ethylene glycol diallyl ether, glycerol diallyl ether, glycerol triallyl ether, polyallyl ethers based on sorbitol, and also ethoxylated variants thereof. The process of the present invention may utilize di(meth)acrylates of polyethylene glycols, the polyethylene glycol used having a molecular weight between 300 and 1000.

However, particularly advantageous crosslinkers b) are di- and triacrylates of 3- to 15-tuply ethoxylated glycerol, of 3- to 15-tuply ethoxylated trimethylolpropane, of 3- to 15-tuply ethoxylated trimethylolethane, especially di- and triacrylates of 2- to 6-tuply ethoxylated glycerol or of 2- to 6-tuply ethoxylated trimethylolpropane, of 3-tuply propoxylated glycerol, of 3-tuply propoxylated trimethylolpropane, and also of 3-tuply mixedly ethoxylated or propoxylated glycerol, of 3-tuply mixedly ethoxylated or propoxylated trimethylolpropane, of 15-tuply ethoxylated glycerol, of 15-tuply ethoxylated trimethylolpropane, of 40-tuply ethoxylated glycerol, of 40-tuply ethoxylated trimethylolethane and also of 40-tuply ethoxylated trimethylolpropane.

Very particularly preferred for use as crosslinkers b) are diacrylated, dimethacrylated, triacrylated or trimethacrylated multiply ethoxylated and/or propoxylated glycerols as described for example in WO 03/104 301 A1. Di- and/or triacrylates of 3- to 10-tuply ethoxylated glycerol are particularly advantageous. Very particular preference is given to di- or triacrylates of 1- to 5-tuply ethoxylated and/or propoxylated glycerol. The triacrylates of 3- to 5-tuply ethoxylated and/or propoxylated glycerol are most preferred. These are notable for particularly low residual contents (typically below 10 weight ppm) in the water-absorbing polymer and the aqueous extracts of the water-absorbing polymers produced therewith have an almost unchanged surface tension (typically at least 0.068 N/m) compared with water at the same temperature.

Examples of ethylenically unsaturated monomers c) which are copolymerizable with the monomers a) are acrylamide, methacrylamide, crotonamide, dimethylaminoethyl methacrylate, dimethylaminoethyl acrylate, dimethylaminopropyl acrylate, diethylaminopropyl acrylate, dimethylaminobutyl acrylate, dimethylaminoethyl methacrylate, diethylaminoethyl methacrylate, dimethylaminoneopentyl acrylate and dimethylaminoneopentyl methacrylate.

Useful water-soluble polymers d) include polyvinyl alcohol, polyvinylpyrrolidone, starch, starch derivatives, polyethyleneimines, polyglycols, polymers formally constructed wholly or partly of vinylamine monomers, such as partially or completely hydrolyzed polyvinylamide (so-called "polyvinylamine") or polyacrylic acids, preferably polyvinyl alcohol and starch.

The polymerisation is optionally carried out in the presence of customary polymerisation regulators. Suitable polymerisation regulators are for example thio compounds, such as thioglycolic acid, mercapto alcohols, for example 2-mercaptoethanol, mercaptopropanol and mercaptobutanol, dodecyl mercaptan, formic acid, ammonia and amines, for example ethanolamine, diethanolamine, triethanolamine, triethylamine, morpholine and piperidine.

The monomers (a), (b) and optionally (c) are (co)polymerized with each other, optionally in the presence of the water-soluble polymers d), in 20% to 80%, preferably 20% to 50% and especially 30% to 45% by weight aqueous solution in the presence of polymerisation initiators. Useful polymerisation initiators include all compounds that disintegrate into free radicals under the polymerisation conditions, examples being peroxides, hydroperoxides, hydrogen peroxide, persulfates, azo compounds and the so-called redox initiators. The use of water-soluble initiators is preferred. It is advantageous in some cases to use mixtures of various polymerisation initiators, examples being mixtures of hydrogen peroxide and sodium or potassium peroxodisulfate. Mixtures of hydrogen peroxide and sodium peroxodisulfate can be used in any desired ratio. Suitable organic peroxides are for example acetylacetone peroxide, methyl ethyl ketone peroxide, tert-butyl hydroperoxide, cumene hydroperoxide, tert-amyl perpivalate, tert-butyl perpivalate, tert-butyl perneohexanoate, tert-butyl perisobutyrate, tert-butyl per-2-ethylhexanoate, tert-butyl perisononanoate, tert-butyl permaleate, tert-butyl perbenzoate, tert-butyl per-3,5,5-trimethylhexanoate and tert-amyl perneodecanoate. Further suitable polymerisation initiators are azo initiators, for example 2,2'-azobis(2-amidinopropane) dihydrochloride, 2,2'-azobis(N,N-dimethylene)-isobutyramidine dihydrochloride, 2-(carbamoylazo)isobutyronitrile and 4,4'-azobis(4-cyanovaleric acid). The polymerisation initiators mentioned are used in customary amounts, for example in amounts of from 0.01 to 5 mol%, preferably 0.1 to 2 mol%, based on the monomers to be polymerized.

The redox initiators comprise, as oxidizing component, at least one of the above-indicated per compounds and a reducing component, for example ascorbic acid, glucose, sorbose, ammonium bisulfite, ammonium sulfite, ammonium thiosulfate, ammonium hyposulfite, ammonium pyrosulfite, ammonium sulfide, alkali metal bisulfite, alkali metal sulfite, alkali metal thiosulfate, alkali metal hyposulfite, alkali metal pyrosulfite, alkali metal sulfide, metal salts, such as iron(II) ions or silver ions, sodium hydroxy-methylsulfoxylate, or sulfinic acid derivatives. The reducing component of the redox initiator is preferably ascorbic acid or sodium pyrosulfite. 1 · 10⁻⁵ to 1 mol% of the reducing component of the redox initiator and 1 · 10⁻⁵ to 5 mol% of the oxidizing component are used based on the amount of monomers used in the polymerisation. Instead of the oxidizing component or in addition it is also possible to use one or more water-soluble azo initiators.

A redox initiator consisting of hydrogen peroxide, sodium peroxodisulfate and ascorbic acid is preferably used. These components are used for example in the concentrations of 1 · 10⁻² mol% of hydrogen peroxide, 0.084 mol% of sodium peroxodisulfate and 2.5 · 10⁻³ mol% of ascorbic acid, based on the monomers.

It is also possible to initiate the polymerisation by the numerous other known means to initiate polymerisations. On example is initiating polymerisation by irradiating with radiation of sufficiently high energy, in particular ultraviolet light. Usually, when initiating polymerisation by ultraviolet light, compounds are added which decompose into radicals upon irradiation by ultraviolet light. Examples of such compunds are 2-hydroxi-2-methyl-1-phenyl-1-propanone and/or alpha,-alpha-dimethoxi-alpha-phenylacetophenone.

The aqueous monomer solution may comprise the initiator in dissolved or dispersed form. However, the initiators may also be added to the kneader used as polymerisation reactor separately from the monomer solution.

The preferred polymerisation inhibitors require dissolved oxygen for optimum effect. Therefore, the polymerisation inhibitors can be freed of dissolved oxygen prior to polymerisation, by inertisation, i.e., by flowing an inert gas, preferably nitrogen, through them. This is accomplished by means of inert gas, which can be introduced concurrently, countercurrently or at entry angles in between. Good commixing can be achieved for example with nozzles, static or dynamic mixers or bubble columns. The oxygen content of the monomer solution is preferably lowered to less than 1 weight ppm and more preferably to less than 0.5 weight ppm prior to polymerisation. The monomer solution is optionally passed through the kneader used as polymerisation reactor using an inert gas stream.

The preparation of a suitable polymer as well as further suitable hydrophilic ethylenically unsaturated monomers a) are described for example in DE 199 41 423 A1, EP 686 650 A1, WO 01/45 758 A1 and WO 03/104 300 A1.

In the process of this invention, an inorganic powder e) is added to the superabsorbent's constituents a) to d) listed above.

In principle, any inorganic powder may be useful therefor. The inorganic powder is a particulate solid. Non-limiting examples of such solids are generally solid, chemically inert (that means, not interfering with the polymerisation reaction in a substantial manner) substances, oxides, zeolithes, inorganic pigments, minerals or clays.

Non-limiting examples of generally suitable inorganic solids are sulphates such as magnesium or barium sulphate, carbonates such as calcium or magnesium carbonate or dolomite, silicates such as calcium or magnesium silicate, carbides such as perlite or silicon carbide, or diatomaceous earth or fly ash.

Suitable oxides are the metal oxides of groups 2 to 14 of the periodic table of the elements including lanthanides and actinides. Non-limiting examples of particularly suitable metal oxides are magnesium oxide, calcium oxide, strontium oxide, barium oxide, titanium dioxide, zirconium dioxide, vanadium oxide, chromium oxide, molybdenum oxide, tungsten oxide, manganese oxide, iron oxide, cobalt oxide nickel oxide, copper oxide, zinc oxide, boron oxide, aluminium oxide (alumina) silicon dioxide (silica), tin oxide, lead oxide, lanthanum oxide or cerium oxide. For clarification purposes: Using common names for individual metal oxides is not intended to make a defining statement about the metal's valence or the stoichiometric composition of the oxide. Whenever an element forms more than one oxide, typically all chemically inert oxides may be used. For any given case, the oxide is chosen according to case-sepcific considerations such as price, toxicity, stability or colour. Non-limiting examples of particularly suitable oxides are titanium dioxide, particularly in the anatase or rutile modifications, or precipitated or fumed silicon dioxide.

Clays are silicate or alumosilicate minerals that typically are obtained by mining and sometimes also further processing natural sediments. Some clays, however, are produced by synthesis.

A clay useful in the present invention can be a swelling or a nonswelling clay. Swelling clays have the ability to absorb water and are swellable, layered inorganic materials. Suitable swelling clays include, but are not limited to, montmorillonite, saponite, nontronite, laponite, beidelite, hectorite, sauconite, stevensite, vermiculite, volkonskoite, magadite, medmontite, kenyaite, and mixtures thereof.

Preferably, the swelling clay is a smectite or vermiculite clay. More preferably, the clay is a smectite clay. Examples of suitable smectites include, but are not limited to, montmorillonite (often just referred to as bentonite, although bentonite technically is a naturally occurring combination of clay particles, rich in montmorillonite that may also include other smectites as well as nonclay mineral constituents, and that exists in swelling (sodium bentonites) or non-swelling (calcium bentonites) forms), beidelite, nontronite, hectorite, saponite, sauconite, and laponite.

Suitable nonswelling clays include, without limitation, kaolin minerals (often just called "kaolin", a naturally occurring combination of clay particles rich in kaolinite, halloysite, dickite and nacrite, that may also include some nonclay mineral constituents), serpentine minerals, mica minerals (including illite), chlorite minerals, sepiolite, palygorskite, bauxite, pyrophyllite, talc, hydrotalcite and mixtures thereof. Kaolin is a preferred inorganic solid.

The clay also can be an organophilic clay. As used here and hereafter, the term "organophilic" is defined as the property of a compound to absorb at least its own weight, and preferably many times its own weight, of an organic, water-immiscible compound. An organophilic compound optionally can absorb water or a water-miscible compound.

The terms "organophilic clay" and "organoclay" are used interchangeably herein to refer to various types of clay, e.g., smectites, that have organoammonium ions substituted for metal cations (e.g., sodium and/or potassium) present between the clay layers. The term "organoammonium ion" refers to a substituted ammonium ion wherein one or more hydrogen atoms are replaced by an aliphatic or aromatic organic group. The organoclays, therefore, are solid compounds that have an inorganic component and an organic component.

The preferred clay substrates of an organophilic clay are the smectite type clays, particularly smectite-type clays that have a cation exchange capacity of at least 75 milliequivalents per 100 grams of clay. Useful clay substrates include, but are not limited to, the naturally occurring Wyoming variety of bentonite and similar clays, and hectorite, which is a magnesium-lithium silicate clay. The clays preferably first are converted to the sodium form if they are not already in this form. This conversion can be effected by a cation exchange reaction using a soluble sodium compound by methods well known in the art. Smectite-type clays prepared synthetically also can be utilized, for example, montmorillonite, bentonite, beidelite, hectorite, saponite, and stevensite.

Other useful clay substrates include nontronite, illite, attapulgite, and a fuller's earth.

It is also possible to use mixtures of two or more of these solid powders.

The inorganic powder is in the form of particles. The average particle size is typically in the range from 0.001 to 500 µm, preferably in the range from 0.002 to 200 µm, more preferably in the range from 0.005 to 100 µm and most preferably in the range from 0.01 to 0.50 µm. The powder particles themselves may be aggregates or agglomerates of smaller primary particles. Particle size may be determined using sieving analysis, but it is easier and therefore preferred to determine particle size using laser diffraction techniques. These are well-known and are routinely performed on dedicated equipment that is commercially available.

The amount of inorganic powder used is typically at least 0.005 wt.-%, preferably at least 0.05 wt.-% and more preferably at least 0.1 wt.-% and typically not more than 20 wt.-%, preferably not more than 10 wt.-% and more preferably not more than 8 wt.-% based on total mass of the final superabsorbent, calculated as having no water or residual moisture content.

According to this invention, superabsorbents are obtained by addition polymerisation of an aqueous monomer solution (or suspension in cases where insoluble constituents such as the inorganic powder are present) and concurring comminution of the polymer-isate in a kneader. Preferably, the process is conducted continuously.

The kneader is equipped with at least two parallel shafts. Preferably, it is equipped with two parallel shafts. The shafts operate in co-rotatory or contra-rotatory fashion, preferably they are contra-rotatory. At least one shaft is and preferably both shafts are equipped with a plurality of elements (or "tools") that knead and also effect an overall transport of the kneader's content parallel to the shafts from a section of entry into the kneader to a discharge section. These elements may be dedicated kneading or transport elements or elements that perform both functions.

Typically, such kneaders are machines of horizontally elongated shape and the casing of two-shaft kneaders resembles two parallel cylinders that partly overlap or a cylinder having an oval rather than circular cross-section, with the kneader's section of entry at one of the kneader's ends (the "upstream end") and the discharge section at the other (the downstream end"). Besides the usual kneading effect that inevitably comes with a certain amount of backmixing, a kneader of this type continuously moves its content along its axis and discharges it at its downstream end. As a reactor, its residence time characteristic thus is between that of an ideal plug flow reactor and that of an ideal fully backmixed stirred tank reactor. A certain point along the kneader's axis thus corresponds to a certain average residence time of its content.

Such kneaders with 2 shafts achieve, by virtue of the arrangement of the kneading and transporting elements, high self-cleaning action, which is an important requirement for a continuous polymerisation. One of the shafts (the "stirring shaft") may designed to effect most of the kneading and transporting functions and the other (the "cleaning shaft") to avoid product-build up on the stirring shaft. The stirring shaft is fitted with disk segments in propeller fashion. Suitable kneading and transporting elements include for example close-clearance mixing bars and L- or U-shaped attachments.

Processes for producing superabsorbent in a kneader as used in this invention, albeit without adding inorganic solids, and kneaders for conducting such processes are taught in WO 03/022 896 A1, WO 01/38 402 A1, WO 2006/034 806 A1 and WO 2006/034 853 A1, or are known in the art and described in other references. Kneaders useful in the process of the present invention are for example the ORP models obtainable from List AG, Arisdorf, Switzerland and those described for example in CH-A 664 704, EP-A 517 068, WO 97/12666, DE-A 21 23 956, EP-A 603 525, DE-A 195 36 944 and DE-A 41 18 884.

In the process of the present invention, the inorganic powder is added prior to or during polymerisation. In other words, the inorganic powder is added either to the monomer solution before this monomer solution ("solution" is used in the context of this invention for the monomer-containing mixture prior to polymerisation, although that mixture technically is a suspension once the inorganic powder has been added) enters the kneader, is fed into the kneader at a place where the kneader's contents have not yet started to polymerise or at a place where some polymerisation has already occurred. It is also possible to add inorganic powder at two or more of such places. The inorganic powder is added to the kneader at a point corresponding to no more than half the average residence time of the kneader's content within the kneader, preferably at a point corresponding to no more than 30 % of the average residence time. Even more preferably, the inorganic powder is added either to the monomer solution fed to the kneader, or is fed to the kneader at a place where the kneader's contents have not started to polymerise, in particular to the feed section. The inorganic powder may be added to the monomer solution fed to the kneader at any suitable position along the monomer solution preparation or feeding system. If a mixing operation under application of mechanical force is performed during monomer preparation or feeding, it may be preferable to add the inorganic powder prior to this mixing step. For example, if the monomer solution is prepared as disclosed in WO 2007/028749 A1 by adding the internal crosslinker to the monomer solution in a Venturi pipe, it may be preferable to to add the inorganic powder to the monomer solution before the solution is fed into the Venturi, preferably directly before the Venturi. In any case, the powder may be added as powder or in the form of a slurry or suspension in a suitable liquid. Conveniently, the liquid is water or any other liquid used in the polymerisation reaction. Examples of such liquids are sodium hydroxide solution, acrylic acid or sodium acrylate solutions.

As the kneader's content are transported through the kneader from its feed section to its discharge section, some backmixing occurs. Consequently, the kneader's content spend a certain average residence time in the kneader. This average residence time depends on the shaft rotating speed and the shaft design. A convenient and well-known method of determining average residence time is starting to add a constant amount of a tracer substance to the feed section during continuous operation of the kneader and monitoring the tracer concentration at the discharge section. This concentration will rise after a certain time as the tracer begins to appear at the discharge section and level out at the added tracer concentration. The average residence time is the time between start of tracer addition and reaching 50 % of the theoretical tracer concentration at the discharge section. Colorants may be used as tracers that are particularly easy to measure. It is generally preferred, however, to use tracers that have no negative impact on the product, and colorants may pose an optical problem for superabsorbents as the customers are used to white products. For superabsorbents, suitable tracer substances comprise potassium hydroxide, calcium chloride, aluminium sulphate or potassium sulphate.

As the kneader's contents are transported through the kneader along its axis, each geometrical position or point along the kneader's axis corresponds to a certain fraction of the average residence time. The kneader may have sections with varying arrangements of kneading and transporting elements on it shafts and correspondingly varying filling levels. Therefore, the travelling speed of the kneader's contents in a direction parallel to the kneader's axis may vary along this axis. In other words, the kneader's contents may need more ore less than a certain fraction of the average residence time to arrive at the same number fraction of the distance between feed and discharge sections. It is easily possible, however, to determine the tracer concentration during a measurement of average residence time at several positions along the kneader's axis, thus determining the average time taken by the kneader's contents to arrive at a certain position in the kneader. In this way, the position along the kneader's axis that corresponds to a defined fraction of the average residence time can easily be determined.

At least one other additive is added to the polymerising mixture in the kneader. These additives is selected from any known additives for superabsorbents. A typical example of another additive are superabsorbent fines. Superabsorbent fines are the undersized particles obtained in classifying operations downstream from drying the as-polymerised gel product. The superabsorbent fines to be added in the process of this invention may be the superabsorbent fines obtained in superabsorbent production downstream of the process of this invention, or fines obtained in another process for producing superabsorbents. Typically, superabsorbent fines are superabsorbent powders (surface-crosslinked or not, or mixtures thereof) having a particle size of not more than 500 µm, even more typically of not more than 300 µm or of not more than 150 µm. As 100 µm or 106 µm are also often used as lower cut-off sizes in superabsorbent particle size specifications, superabsorbent fines often have particle sizes of less than 106 or less than 100 µm. A typical minimum particle size is 20 µm as smaller particles may form airborne dust and pose a respiratory hazard, although it is perfectly possible to use such fines in the process of this invention.

The superabsorbent fines are added to the polymerising mixture in the kneader at a position corresponding to at least half the average residence time, or in other words at a position corresponding to no earlier than half the average residence time. The fines may be added at several positions, provided each of them fulfils this condition. In one embodiment, the fines are added no earlier than at a position corresponding to 60 % of the residence time.

Superabsorbent fines are generally added "as is", that means as they are obtained in downstream classifying operations (typically sieving or sifting). They may be pretreated to improve handling properties prior to recycling them into the polymerisation step according to this invention. A suitable pre-treatment may be moistening the fines to feed them to the kneader as a gel rather than as powder.

Prior to polymerisation, the monomer solution is freed of residual oxygen. This is accomplished by means of an inert gas, which may be introduced in cocurrent, in countercurrent or at intermediate entry angles. Good mixing may be obtained for example using nozzles, static or dynamic mixers or bubble columns.

The monomer solution is passed through the kneader with or without an inert gas stream. In production-scale reactors, the mass throughput in terms of monomer solution is preferably not less than 500 kg/hm³, more preferably not less than 1000 kg/hm³, even more preferably not less than 2000 kg/hm³ and especially not less than 3000 kg/hm³ (kneader volume) with the inert gas stream, if applied, preferably being not less than 100 I/hm³ (kneader volume). Of course, throughput and volume of pilot-plant-sized or laboratory reactors may be considerably smaller.

The inert gases used may each independently be steam, nitrogen, a noble gas such as argon, carbon monoxide, carbon dioxide, sulfur hexafluoride or a mixture thereof. The inert gas may be wholly or partly generated by a chemical reaction in the kneader. Preference is given to using steam, carbon dioxide and/or nitrogen as inert gas.

The kneader volume may vary according to the desired throughput and conversion. The kneader volume is preferably not less than 0.5 m³ more preferably at least 0.7 m³, even more preferably in the range from 1 to 25 m³ and especially in the range from 3 to 12 m³.

While the mixture fed into the kneader has comparatively low viscosity its consistency changes via a highly viscous state into a crumbly gel which is discharged at the downstream end of the kneader by the continuous conveying action of the kneader. The gel produced by the polymerisation is comminuted into a finely divided, crumbly gel in the kneader and is then discharged in that state. Preferably, some of the water is removed during the polymerisation in the mixer, so that crumbly particles of gel obtained at the downstream end of the kneader have a solids content in the range from 20% to 100% by weight.

Preferably, the fill level in the kneader, defined and measured in the region of crumbly gel as described in WO 2006/034 806 A1, is not less than 71 % and preferably not more than 99% and more preferably is in the range between 73% and 95% and even more preferably in the range between 75% and 90% and especially in the range from 80% to 85%. Further, the temperature in the polymerisation zone as defined and described in WO 2006/034 806 A1 preferably is more than 65°C, preferably more than 70°C, more preferably more than 75°C, even more preferably more than 80°C and especially more than 85°C. The upper limit of the temperature in the polymerisation zone is generally at 100°C, preferably at 96°C. In a particularly preferred embodiment, the temperature fluctuations per hour are below 20°C, preferably 15°C, more preferably 10°C, especially 5°C. The temperature and the temperature fluctuation can be brought into this range by a high fill level of at least 71 %, by heating or cooling, or by metered addition of fine particles of the superabsorbent, typically undersized product particles obtained in downstream sieving operations.

Average residence time and residence time distribution (the latter may be described as maximum deviation from average residence time) of the kneader's contents within the kneader are chosen according to the individual requirements for the pertinent plant in terms of yield and throughput. Process and kneader design features that set these parameters include the kneader's internal volume, the monomer solution feed rate, the kneader fill level and the design of the kneader shafts (including the kneading and transport elements) in that the shaft geometry will create a more or less pronounced backward or forward transportation of the material, thus determining the degree of backmixing, expressed as backmixing ratio (the backmixing ratio is the quotient of residence time distribution and mean residence time. The degree of backmixing may further be influenced via variation of the fill height (weir at kneader outlet) and also metering rate at kneader inlet or at various locations in the kneader (various possible additives) and also changes in the speed of rotation of the stirring shafts and specific back-conveying zones.

When a broad residence time distribution is preferred for reasons of quality, for example in order to smooth out quality fluctuations in the material produced, kneaders having a broad residence time distribution will typically be chosen.

In many cases, however, a backmixing ratio, measured as described in WO 2006/034 806 A1, of less than 0.33 is applied, preferably less than 0.3, more preferably less than 0.27, even more preferably less than 0.26 or even less than 0.25, especially less than 0.24.

Appropriate process and kneader design is known. Typically, average residence time is minimised to optimize space-time yield.

The reaction may also be carried out under reduced pressure at 100 - 800 mbar and especially in the range from 200 to 600 mbar.

The kneader may be heated or cooled as required. The monomer solution is polymerized therein at a temperature in the range from -10°C and preferably 0°C to 140°C and preferably 100°C. The temperature is preferably in the range from 30 to 120°C and especially the maximum temperature is in the range from 50 to 100°C, more preferably not more than 95°C and especially not more than 90°C.

The process of the present invention is preferably carried out such that the fraction of heat removed by evaporation of water from the reaction mixture is not less than 5%, preferably not less than 15% and more preferably not less than 25% of the heat of reaction.

Preference is further given to versions of the process in which the fraction of heat removed by product discharge is not less than 25%, preferably not less than 45% and especially not less than 55% of the heat of reaction.

Preference is given to processes in which in total not less than 50%, more preferably not less than 70% and especially not less than 90% of the heat of reaction is removed by product discharge and water evaporation.

In a very particularly preferred process variant, the inner surface of the kneader and/or one or more, preferably all, shafts are cooled.

The gel obtained in the polymerisation has a water content in the range from 0% to 80% by weight and preferably in the range from 40% to 70% by weight. This relatively low moisture content for an already free-flowing gel which does not clump reduces the energy subsequently required for drying.

The gel obtained in the polymerisation typically has a residual monomer content of below 170 ppm, preferably 160 ppm or less. Even values of 150 ppm or less, 120 ppm or less and even 100 ppm or less can be achieved with the process of the present invention.

The time taken to attain peak temperature in the process of the present invention is preferably 5 minutes or less and more preferably in the range from 2 to 4 minutes. This range includes the optimum with regard to throughput in the kneader and product quality (few agglomerates, good residual monomer values, etc.).

Details of process operation are described in the cited references.

The acid groups of the hydrogels obtained are partially neutralized in an acid neutralisation step, generally to an extent of at least 25 mol%, preferably to an extent of at least 50 mol% and more preferably at least 60 mol% and generally to an extent of not more than 85 mol%, preferably not more than 80 mol%, and more preferably not more than 75 mol%.

Neutralisation can be carried out after polymerisation, at the hydrogel stage. But it is also possible to carry out the neutralisation to the desired degree of neutralisation wholly or partly prior to polymerisation. In the case of partial neutralisation and prior to polymerisation, generally at least 10 mol%, preferably at least 15 mol% and also generally not more than 40 mol%, preferably not more than 30 mol% and more preferably not more than 25 mol% of the acid groups in the monomers used are neutralized prior to polymerisation by adding a portion of the neutralizing agent to the monomer solution. The desired final degree of neutralisation is in this case only set toward the end or after the polymerisation, preferably at the level of the hydrogel prior to its drying. The monomer solution is neutralized by admixing the neutralizing agent. The hydrogel can be mechanically comminuted in the course of the neutralisation, for example by means of a meat grinder or comparable apparatus for comminuting gellike masses, in which case the neutralizing agent can be sprayed, sprinkled or poured on and then carefully mixed in. To this end, the gel mass obtained can be repeatedly meat-grindered for homogenisation.

Neutralisation of the monomer solution to the desired final degree of neutralisation prior to polymerisation by addition of the neutralizing agent or conducting the neutralisation after polymerisation is usually simpler than neutralisation partly prior to and partly after polymerisation and therefore is preferred.

The as-polymerized gels are optionally maintained for some time, for example for at least 30 minutes, preferably at least 60 minutes and more preferably at least 90 minutes and also generally not more than 12 hours, preferably for not more than 8 hours and more preferably for not more than 6 hours at a temperature of generally at least 50°C and preferably at least 70°C and also generally not more than 130°C and preferably not more than 100°C, which further improves their properties in many cases. This may be done in a final section of the kneader or in a separate piece of equipment downstream from the kneader.

The neutralized hydrogel is then dried with a belt or drum dryer until the residual moisture content is typically below 15% by weight, especially below 10% by weight and most preferably below 5 wt.-%, the water content being determined by as described below. Optionally, however, drying can also be carried out using a fluidized bed dryer or a heated ploughshare mixer. To obtain particularly colourless products, it is advantageous to dry this gel by ensuring rapid removal of the evaporating water. To this end, dryer temperature must be optimized, air feed and removal has to be policed, and at all times sufficient venting has to be ensured. Drying is naturally all the more simple - and the product all the more colourless - when the solids content of the gel is as high as possible. The solvent fraction at addition polymerisation is therefore set such that the solid content of the gel prior to drying is therefore generally at least 20% by weight, preferably at least 25% by weight and more preferably at least 30% by weight and also generally not more than 90% by weight, preferably not more than 85% by weight and more preferably not more than 80% by weight. It is particularly advantageous to vent the dryer with nitrogen or some other non-oxidizing inert gas. Optionally, however, simply just the partial pressure of oxygen can be lowered during drying to prevent oxidative yellowing processes. But in general adequate venting and removal of the water vapour will likewise still lead to an acceptable product. A very short drying time is generally advantageous with regard to colour and product quality.

The dried hydrogel (which is no longer a gel (even though often still called that) but a dry polymer having superabsorbing properties, which comes within the term "superabsorbent") is preferably ground and sieved, useful grinding apparatus typically including roll mills, pin mills, hammer mills, cutting mills or swing mills. The particle size of the sieved, dry hydrogel is preferably below 1000 µm, more preferably below 900 µm and most preferably below 850 µm and preferably above 80 µm, more preferably above 90 µm and most preferably above 100 µm. The undersized particles are superabsorbent fines than may be recycled into the polymerisation step according to the process of this invention.

Very particular preference is given to a particle size (sieve cut) in the range from 106 to 850 µm. Particle size (or rather, particle size distribution) is determined as described below.

The dry superabsorbing polymers thus produced are typically known as "base polymers" and are then surface postcrosslinked. Surface postcrosslinking can be accomplished in a conventional manner using dried, ground and classified polymeric particles. For surface postcrosslinking, compounds capable of reacting with the functional groups of the base polymer by crosslinking are applied, usually in the form of a solution, to the surface of the base polymer particles. Usually, a surface crosslinker solution is first applied to the base polymer by contacting base polymer and crosslinker solution, and then the formation of surface crosslinks is effected or completed by heat treatment. The contacting step leads to a coating of surface crosslinking solution on the base polymer particles, possibly to some crosslinking depending on reactivity of the crosslinker and temperature applied during the contacting step, and the heat treatment step to a finished surface-crosslinked superabsorbent.

Contacting the base polymer with a surface postcrosslinking solution (again, "surface crosslinking" is used synonymously) is typically carried out by spraying the surface postcrosslinking solution of the surface postcrosslinker ("surface crosslinker") onto the hydrogel or the dry base polymer powder.

Suitable organic postcrosslinking agents are for example:
- di- or polyepoxides, for example di- or polyglycidyl compounds such as phosphonic acid diglycidyl ether, ethylene glycol diglycidyl ether, bischlorohydrin ethers of polyalkylene glycols,
- alkoxysilyl compounds,
- polyaziridines, compounds comprising aziridine units and based on polyethers or substituted hydrocarbons, for example bis-N-aziridinomethane,
- polyamines or polyamidoamines and also their reaction products with epichlorohydrin,
- polyols such as ethylene glycol, 1,2-propanediol, 1,4-butanediol, glycerol, methyltriglycol, polyethylene glycols having an average molecular weight Mw of 200 - 10 000, di- and polyglycerol, pentaerythritol, sorbitol, the ethoxylates of these polyols and also their esters with carboxylic acids or carbonic acid such as ethylene carbonate or propylene carbonate,
- carbonic acid derivatives such as urea, thiourea, guanidine, dicyandiamide, 2-oxazolidinone and its derivatives, bisoxazoline, polyoxazolines, di- and polyisocyanates,
- di- and poly-N-methylol compounds such as for example methylenebis(N-methylolmethacrylamide) or melamine-formaldehyde resins,
- compounds having two or more blocked isocyanate groups such as for example trimethylhexamethylene diisocyanate blocked with 2,2,3,6-tetramethylpiperidin-4-one.

If necessary, acidic catalysts can be added, examples being p-toluenesulfonic acid, phosphoric acid, boric acid or ammonium dihydrogenphosphate.

Particularly suitable postcrosslinking agents are di- or polyglycidyl compounds such as ethylene glycol diglycidyl ether, the reaction products of polyamidoamines with epichlorohydrin, 2-oxazolidinone and N-hydroxyethyl-2-oxazolidinone.

The solvent used for the surface postcrosslinker is a customary suitable solvent, examples being water, alcohols, DMF, DMSO and also mixtures thereof. Examples of suitable alcohols are monools, diols, triols or polyols, preferably of alcohols having one to eight carbon atoms. Preferred are the propanoles. Most preferably, the alcohol is selected from the group consisting of propylene glycol, 1,3-propandiol, 1-propanol, 2-propanol and mixtures thereof. Particular preference is given to water and water-alcohol mixtures, examples being water-methanol, water-1,2-propanediol, water-2-propanol and water-1,3-propanediol.

The spraying with a solution of the postcrosslinker is preferably carried out in mixers having moving mixing implements, such as screw mixers, paddle mixers, disk mixers, plowshare mixers and shovel mixers. Particular preference is given to vertical mixers and very particular preference to plowshare mixers and shovel mixers. Useful and known mixers include for example Lödige^{®}, Bepex^{®}, Nauta^{®}, Processall^{®} and Schugi^{®} mixers. Very particular preference is given to high speed mixers, for example of the Schugi-Flexomix^{®} or Turbolizer^{®} type.

The polymer is optionally further surface crosslinked using a polyvalent metal salt. Preferably, the polyvalent metal salt is water-soluble. Water-soluble polyvalent metal salts comprise bi- or more highly valent ("polyvalent") metal cations capable of reacting with the acid groups of the polymer to form complexes. Examples of polyvalent cations are metal cations such as Mg²⁺, Ca²⁺, Al³⁺, Sc³⁺, Ti⁴⁺, Mn²⁺, Fe^{2+/3+}, Co²⁺, Ni²⁺, Cu²⁺, Zn²⁺, Y³⁺, Zr⁴⁺, La³⁺, Ce⁴⁺, Hf⁴⁺, and Au³⁺. Preferred metal cations are Mg²⁺, Ca²⁺, Al³⁺, Ti⁴⁺, Zr⁴⁺ and La³⁺, and particularly preferred metal cations are Al³⁺, Ti⁴⁺ and Zr⁴⁺. Most preferred is Al³⁺. The metal cations can be used not only alone but also in admixture with each other. Of the metal cations mentioned, any metal salt can be used that has sufficient solubility in the solvent to be used. Metal salts with weakly complexing anions such as for example chloride, nitrate and sulphate, hydrogen sulphate, carbonate, hydrogen carbonate, nitrate, phosphate, hydrogen phosphate, dihydrogen phosphate and carboxylate, such as acetate and lactate, are particularly suitable. It is particularly preferred to use aluminium sulfate.

The superabsorbent is contacted with the polyvalent metal salt generally in the form of a polyvalent metal salt solution. Examples of suitable solvents are water, alcohols DMF, DMSO and also mixtures thereof. Examples of suitable alcohols are monools, diols, triols or polyols, preferably of alcohols having one to eight carbon atoms. Preferred are the propanoles. Most preferably, the alcohol is selected from the group consisting of propylene glycol, 1,3-propandiol, 1-propanol, 2-propanol and mixtures thereof. Particular preference is given to water and water-alcohol mixtures such as for example water-methanol, water-1,2-propanediol, water-2-propanol and water-1,3-propanediol.

Contacting the superabsorbent polymer with solution of a polyvalent cation is carried out in the same way as that described above for organic surface postcrosslinker.

Generally, the amount of organic crosslinker applied to the superabsorbent is at least 50 wt.-ppm, preferably at least 100 wt.-ppm, more preferably at least 200 wt.-ppm and generally not more than 1 wt.-%, preferably not more than 0,5 wt.-% and more preferably not more than 2000 wt.-ppm, based on the weight of the base polymer. The amount of polyvalent metal salt applied is generally at least 50 wt.-ppm, preferably 1000 wt.-ppm and more preferably at least 2000 wt.-ppm and generally not more than 5 wt.-%, preferably not more than 3 wt.-% and more preferably not more than 1 wt.-%, based on the weight of the base polymer. The amount of alcohol applied is generally at least 1000 wt.-ppm, preferably at least 2000 wt.-ppm and more preferably at least 3000 wt.-% and generally not more than 15 wt.-%, preferably not more than 10 wt.-% and more preferably not more than 5 wt.-%, based on the weight of base polymer.

The step of contacting a superabsorbent base polymer with an organic crosslinker and a polyvalent metal salt solution can be optionally, and preferably is, followed by a thermal treatment step, essentially to effect the surface-postcrosslinking reaction (yet usually just referred to as "drying"), preferably in a downstream heated mixer ("dryer") at a temperature of generally at least 50°C, preferably at least 80°C and more preferably at least 80°C and also generally not more than 300°C, preferably not more than 250°C and more preferably not more than 200°C. The average residence time (i.e., the averaged residence time of the individual particles of superabsorbent) in the dryer of the superabsorbent to be treated is generally at least 1 minute, preferably at least 3 minutes and more preferably at least 5 minutes and also generally not more than 6 hours, preferably 2 hours and more preferably not more than 1 hour. As well as the actual drying taking place, not only any products of scissioning present but also solvent fractions are removed. Thermal drying is carried out in customary dryers such as tray dryers, rotary tube ovens or heatable screws, preferably in contact dryers. Preference is given to the use of dryers in which the product is agitated, i.e., heated mixers, more preferably shovel dryers and most preferably disk dryers. Bepex^{®} dryers and Nara^{®} dryers are suitable dryers for example. Fluidized bed dryers can also be used. But drying can also take place in the mixer itself, by heating the jacket or blowing a preheated gas such as air into it. But it is also possible for example to utilize an azeotropic distillation as a drying process. The crosslinking reaction can take place not only before but also during drying.

When water is present in the base polymer or organic crosslinker solution, it is preferred to conduct the heat treatment at conditions sufficient to reduce the moisture content of the resulting surface-crosslinked superabsorbent to a value of less than 1 wt.%, based on the total amount of surface-crosslinked superabsorbent.

After any drying or heat treatment step, it is advantageous but not absolutely necessary to cool the product after drying. Cooling can be carried out continuously or discontinuously, conveniently by conveying the product continuously into a cooler downstream of the dryer. Any apparatus known for removing heat from pulverulent solids can be used, in particular any apparatus mentioned above as a drying apparatus, provided it is supplied not with a heating medium but with a cooling medium such as for example with cooling water, so that heat is not introduced into the superabsorbent via the walls and, depending on the design, also via the stirrer elements or other heat-exchanging surfaces, but removed from the superabsorbent. Preference is given to the use of coolers in which the product is agitated, i.e., cooled mixers, for example shovel coolers, disk coolers or paddle coolers, for example Nara^{®} or Bepex^{®} coolers. The superabsorbent can also be cooled in a fluidized bed by blowing a cooled gas such as cold air into it. The cooling conditions are set such that a superabsorbent having the temperature desired for further processing is obtained. Typically, the average residence time in the cooler will be in general at least 1 minute, preferably at least 3 minutes and more preferably at least 5 minutes and also in general not more than 6 hours, preferably 2 hours and more preferably not more than 1 hour, and cooling performance will be determined such that the product obtained has a temperature of generally at least 0°C, preferably at least 10°C and more preferably at least 20°C and also generally not more than 100°C, preferably not more than 80°C and more preferably not more than 60°C.

Optionally, the superabsorbent is provided with further customary additives and auxiliary materials to influence storage or handling properties. Examples thereof are permeability enhancing agents other than surface crosslinkers, such as particulate solids (silica is widely used) or cationic polymers to further enhance permeability, colorations, opaque additions to improve the visibility of swollen gel, which is desirable in some applications, surfactants, cohesion control agents to improve flowability, water to re-moisturise the superabsorbent, or the like. These additives and auxiliary materials can each be added in separate processing steps by methods generally known in the art, but one convenient method may be to add them to the superabsorbent in the cooler, for example by spraying the superabsorbent with a solution or adding them in finely divided solid or in liquid form, if this cooler provides sufficient mixing quality.

The final surface-crosslinked superabsorbent is optionally ground and/or sieved in a conventional manner. Grinding is typically not necessary, but the sieving out of agglomerates which are formed or undersize is usually advisable to set the desired particle size distribution for the product. Agglomerates and undersized particles are either discarded or preferably returned into the process in a conventional manner and at a suitable point; agglomerates after comminution, and the undersized particles are superabsorbent fines that may be recycled into the polymerisation step according to the process of this invention. The superabsorbent particle size is preferably not more than 1000 µm, more preferably not more than 900 µm, most preferably not more than 850 µm, and preferably at least 80 µm, more preferably at least 90 µm and most preferably at least 100 µm. Typical sieve cuts are for example 106 to 850 µm or 150 to 850 µm.

The superabsorbent thus obtained may be used for any use known for superabsorbents, and be processed using any known methods. In particular, it may be used in hygiene products as is known in the art.

### Superabsorbent Property Test Methods

The "WSP" test methods referred to below are described in "Standard Test Methods for the Nonwovens Industry", 2005 edition, jointly published by "Worldwide Strategic Partners" EDANA (European Disposables and Nonwovens Association, Avenue Eugène Plasky, 157, 1030 Brussels, Belgium, www.edana.org) and INDA (Association of the Nonwoven Fabrics Industry, 1100 Crescent Green, Suite 115, Cary, North Carolina 27518, U.S.A., www.inda.org). The publication is available from either EDANA or INDA.

### Centrifuge Retention Capacity (CRC)

Centrifuge Retention Capacity (CRC) is determined using Standard Test Method WSP 241.2 (05).

### Absorption Under Load 0.7 psi (AUL 0.7 psi)

Absorption Under Load 0.7 psi (AUL 0.7 psi) is determined using Standard Test Method WSP 242.2 (05) with the following modification: A plastic piston and cylindrical weight with a total weight of 1347 grams instead of 527 grams are used to obtain a pressure of 0.7 psi.

### Particle Size Distribution (PSD)

Particle Size Distribution is determined using Standard Test Method WSP 220.2 (05).

### Moisture Content

Water or Moisture Content is determined using Standard Test Method WSP 230.2 (05).

### Free Swell Gel Bed Permeability (Free Swell GBP)

The method for determination of the Free Swell Gel Bed Permeability is described in US patent application no. US 2005/0 256 757 A1, paragraphs [0061] through [0075].

### Examples

In the following, feeding or adding "at a x % position" means that the pertinent component is fed or added into the kneader to be used according to the invention through an inlet positioned at a point where the kneader's content has spent, on average, x % of its total average residence time in the kneader. As the kneader's content are force-fed through the kneader by the transporting elements on the kneading shafts, this time corresponds to a particular annular region of the kneader. The 0 % position is the monomer inlet, the 100 % position the product discharge. Similarly, adding "at a x % time", as used in the comparative experiments in a conventional, non-transporting batch kneader means that the pertinent component is added at x % of the total polymerisation time.

"Clay loss", as an indication of the quality of clay incorporation into the polymer matrix, is defined in the following as clay content of the < 106 µm sieve cut of the superabsorbents obtained divided by the clay content of the 106-850 µm sieve cut. The higher the number, the more clay is lost in the fines instead of being incorporated into the product put to use.

### Example 1 (comparative): Polymer A

An aqueous monomer solution was prepared consisting of
9.18 wt.-% of acrylic acid;
81.75 wt.-% of a 37.7 wt.-% aqueous sodium acrylate solution;
8.99 wt.-% of deionised water;
0.08 wt.-% of the triacrylate of 3-tuply ethoxilated glycerol (Laromer® 9044V, obtained from BASF Aktiengesellschaft, Ludwigshafen, Germany).

Oxygen was removed by stripping with a nitrogen stream while stirring at a temperature of 35 °C. The monomer solution was then fed into a continuous two-shaft kneader with a stirring and a cleaning shaft of 10 I volume (model ORP 10 obtained from List AG, Arisdorf, Switzerland, modified for continuous operation mode) at a rate of 21 kg/h. The kneader's jacket was kept at 80 °C. The rotation speed of the cleaning shaft was 50 rpm, the rotation of the stirring shaft was 14 rpm. 10.3 g/h of sodium persulphate, 11.4 g/h of a 3 wt.-% aqueous hydrogen peroxide solution and 0.69 g/h of ascorbic acid was fed separately into the kneader to initiate polymerisation. 550 g/h of Hysorb^{®} B 7055 fines (a < 150 µm sieve cut of Hysorb^{®} B7055 superabsorbent, available from BASF SE, Ludwigshafen, Germany) were fed into the kneader at the 50 % position. A white gel was obtained at the outlet of the kneader that was dried batchwise in a drying oven at 180 °C for 60 minutes, ground and sieved to obtain a 106-850 µm sieve cut.

250 g of the dry polymer thus obtained was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer A.

The properties of polymer A are summarised in table 1.

### Example 2: Polymer B

Example 1 was repeated, however, the monomer solution consisted of
9.18 wt.-% of acrylic acid;
81.75 wt.-% of a 37.7 wt.-% aqueous sodium acrylate solution;
2,34 wt.-% of kaolin clay slurry (70 wt.-% aqueous hydrated alumina silicate suspension, type Ultra White^{®} 90 Slurry obtained from Engelhard Corp. [now supplied by BASF Corporation, Florham Park, New Jersey, U.S.A.])
6.65 wt.-% of deionised water;
0.08 wt.-% of the triacrylate of 3-tuply ethoxilated glycerol (Laromer® 9044V, obtained from BASF Aktiengesellschaft, Ludwigshafen, Germany).

A white gel was obtained at the outlet of the kneader that was dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (Kaolin clay calculated as Al₂Si₂O₅(OH)₄).

### Results:

| Particle size cut | Aluminium content | Clay content |
|---|---|---|
| < 106 µm | 1.44 wt.-% | 4.8 wt.-% |
| 106 - 850 µm | 1.14 wt.-% | 3.8 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer B.

The properties of polymer B are summarised in table 1.

### Example 3: Polymer C

Example 1 was repeated, however, 491.8 g/h of kaolin clay slurry (Ultra White^{®} 90) were fed as a separate stream into the kneader at the 30 % position.

A white gel was obtained at the outlet of the kneader that was dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 1.58 wt.-% | 5.3 wt.-% |
| 106 - 850 µm | 1.11 wt.-% | 3.7 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer C.

The properties of polymer C are summarised in table 1.

### Example 4 (comparative): Polymer D

A 5700 g batch of aqueous monomer solution was prepared from
523 g of acrylic acid;
4660 g of a 37.7 wt.-% aqueous sodium acrylate solution;
530.7 g of deionised water;
   and
4.3 g of the triacrylate of 3-tuply ethoxilated glycerol (Laromer® 9044V, obtained from BASF Aktiengesellschaft, Ludwigshafen, Germany).

Oxygen was removed by stripping with a nitrogen stream while stirring. The batch was then transferred into a jacketed, twin-arm batch kneader of 10 I volume with two sigma-type blades (model HKS 10 obtained from IKA^{®} Werke GmbH & Co. KG, Staufen, Germany). Temperature was equilibrated by letting the batch stand while the kneader's jacket was kept at 35 °C. Rotation of the two arms of the kneader reactor was started as soon as the kneader was filled with the monomer solution at rotation speeds of 70 rpm and 42 rpm, respectively. After that, 2.8 g sodium persulphate, 3.1 g of a 3 wt.-% aqueous hydrogen peroxide solution and 0.187 g of ascorbic acid were added to initiate polymerisation. The kneader's jacket temperature was raised to 80 °C and the speed of the arms was reduced to 50 rpm and 30 rpm, respectively. The batch was allowed to polymerise for 9 minutes at these conditions, then 149.5 g of Hysorb^{®} B 7055 fines were added to the kneader and the polymerisation was continued for 6 more minutes. The obtained gel was then kept in the kneader at 80 °C for 15 more minutes without kneading. The gel was removed from the kneader and dried in a drying oven at 180 °C for 60 minutes, ground and sieved to obtain a 106-850 µm sieve cut.

250 g of the dry polymer was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer D.

The properties of polymer D are summarised in table 1.

### Example 5 (comparative): Polymer E

Example 4 was repeated, however, 133.5 g of kaolin clay slurry (Ultra White^{®} 90) were added to the monomer mixture batch prior to adding the initiator mixture. The gel was removed from the kneader after the polymerization reaction and dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 3.02 wt.-% | 10.1 wt.-% |
| 106 - 850 µm | 0.84 wt.-% | 2.8 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer E.

The properties of polymer E are summarised in table 1.

### Example 6 (comparative): Polymer F

Example 4 was repeated, however, 133.5 g of kaolin clay slurry (Ultra White^{®} 90) were added to the polymerising mixture 5 minutes after adding the initiator mixture. The gel was removed from the kneader after the polymerization reaction and dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 3.80 wt.-% | 12.7 wt.-% |
| 106 - 850 µm | 0.72 wt.-% | 2.4 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer F.

The properties of polymer F are summarised in table 1.

### Example 7 (comparative) Polymer G

Example 1 was repeated, however, 491.8 g/h of kaolin clay slurry (Ultra White^{®} 90) and 550 g/h of HySorb^{®} B 7055 fines were separately fed into the kneader at the 30 % position.

A white gel was obtained at the outlet of the kneader that was dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 1.93 wt.-% | 6.4 wt.-% |
| 106 - 850 µm | 1.05 wt.-% | 3.5 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer G.

The properties of polymer G are summarised in table 1.

### Example 8 (comparative): Polymer H

Example 1 was repeated, however, 491.8 g/h of kaolin clay slurry (Ultra White^{®} 90) and 550 g/h of HySorb^{®} B 7055 fines < 150 µm were fed as separate streams into the kneader at the 60% position.

A white gel was obtained at the outlet of the kneader that was dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 4.47 wt.-% | 14.9 wt.-% |
| 106 - 850 µm | 0.60 wt.-% | 2.0 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer H.

The properties of polymer H are summarised in table 1.

### Example 9 (comparative): Polymer J

Example 1 was repeated, however, 550 g/h of HySorb^{®} B 7055 fines < 150 µm were fed into the kneader at the 30% position and 491.8 g/h of kaolin clay slurry (Ultra White^{®} 90) were fed into the kneader at the 60% position.

A white gel was obtained at the outlet of the kneader that was dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 4.13 wt.-% | 13.8 wt.-% |
| 106 - 850 µm | 0.66 wt.-% | 2.2 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer J.

The properties of polymer J are summarised in table 1.

### Example 10 (comparative): Polymer K

Example 2 was repeated, however, 550 g/h of HySorb^{®} B 7055 fines < 150 µm were fed into the kneader at the 30% position.

A white gel was obtained at the outlet of the kneader that was dried in a drying oven at 180 °C for 60 minutes, ground and sieved to obtain a sieve cut < 106 µm and a 106-850 µm sieve cut. The aluminium content of the material of the two sieve cuts were determined by Atomic Absorption Spectroscopy in order to calculate the clay content of the material (kaolin clay calculated as Al₂Si₂O₅(OH)₄).

### Results:

| Particle size cut | Aluminium content | Clay content |
|---|---|---|
| < 106 µm | 1.59 wt.-% | 5.3 wt.-% |
| 106 - 850 µm | 1.11 wt.-% | 3.7 wt.-% |

250 g of the dry polymer of the 106 - 850 µm sieve cut was put into a food processor and, while stirring, sprayed with a solution of 0.1 wt.-% of ethylene glycol diglycidyl ether, 1 wt.-% of 1,2-propane diol and 2 wt.-% water (amounts based on weight of the polymer powder prior to treatment) via an atomizer nozzle during 3 minutes. Stirring was continued for further 2 minutes. The polymer was then re-dried at 140 °C for 40 minutes to obtain polymer K.

The properties of polymer K are summarised in table 1.

**Table 1**

| Polymer | Kaolin added at x % position/time | Fines added at x % position/time | Clay loss | CRC | AUL 0.7 psi | Free Swell GBP |
|---|---|---|---|---|---|---|
| | | | | [g/g] | [g/g] | [10⁻⁷ cm³s/g] |
| A^{*)} | - | 50 | - | 31.0 | 25.1 | 17 |
| B | 0 | 50 | 1.3 | 29.3 | 23.7 | 23 |
| C | 30 | 50 | 1.4 | 29.6 | 24.0 | 48 |
| D^{*)} | - | 60 | - | 31.3 | 24.5 | 15 |
| E^{*)} | 0 | 60 | 3.6 | 29.5 | 22.8 | 16 |
| F^{*)} | 30 | 60 | 5.3 | 30.4 | 23.9 | 15 |
| G^{*)} | 30 | 30 | 1.8 | 26.5 | 21.3 | 35 |
| H^{*)} | 60 | 60 | 7.5 | 29.8 | 23.9 | 28 |
| J^{*)} | 60 | 30 | 6.3 | 26.3 | 21.1 | 30 |
| K^{*)} | 0 | 30 | 1.4 | 26.1 | 20.9 | 26 |

| | | | | | | |
|---|---|---|---|---|---|---|
| ^{*)} comparative | | | | | | |

The examples firstly demonstrate that using the "transporting" kneader according to the invention enhances the product's Free Swell GBP even without adding inorganic solids (compare polymer A to polymer D) and secondly that adding inorganic powder improves free swell GBP (compare polymer B or C to A; or polymer E or F to D). The examples further demonstrate that addition of inorganic powder at a < 50% position improves incorporation of the inorganic powder into the polymer matrix and that addition of superabsorbent fines at a >50% position maintains a high CRC value. The examples also demonstrate that adding inorganic powder and superabsorbent fines in the "transporting" kneader enhances Free Swell GBP much more than could be expected from the combined effects of either measure alone.

## Claims

1. A process for producing superabsorbents by polymerisation in a kneader equipped with at least two parallel shafts that also comprises elements on at least one shaft that transport the kneader's content parallel to the shafts from a feed section to a discharge section, the process comprising the addition of an inorganic powder to the kneader's contents prior to or during polymerisation and the addition of at least one other additive to the kneader's contents during polymerisation, wherein the inorganic powder is added no later than half the average residence time of the kneader's contents and the other additive is added no earlier than half this residence time.

2. The process of claim 1, wherein the inorganic powder is added to the monomer solution fed to the kneader.

3. The process of claim 1, wherein the inorganic powder is added to the kneader's feed section.

4. The process of claim 1, wherein the inorganic powder is added no later than 30 % of the average residence time of the kneader's contents.

5. The process of any of claims 1 to 4, wherein the inorganic powder is a clay.

6. The process of claim 5, wherein the inorganic powder is kaolin.

7. The process of any of claims 1 to 6, wherein the other additive are superabsorbent fines.

## Patentansprüche

1. Verfahren zum Herstellen von Superabsorptionsmitteln durch Polymerisation in einem Kneter, der mit wenigstens zwei parallelen Wellen ausgestattet ist und ferner Elemente an wenigstens einer Welle aufweist, die den Inhalt des Kneters parallel zu den Wellen von einem Zufuhrabschnitt zu einem Ausstoßabschnitt transportieren, wobei das Verfahren das Zugeben eines anorganischen Pulvers zu dem Inhalt des Kneters vor oder während der Polymerisation und das Zugeben von wenigstens einem anderen Zusatzstoff zu dem Inhalt des Kneters während der Polymerisation umfasst, wobei das anorganische Pulver nicht später als bei der Hälfte der mittleren Aufenthaltszeit des Inhalts des Kneters zugegeben wird und der andere Zusatzstoff nicht früher als bei der Hälfte dieser Aufenthaltszeit zugegeben wird.

2. Verfahren gemäß Anspruch 1, wobei das anorganische Pulver zu der Monomerlösung, die dem Kneter zugeführt wird, zugegeben wird.

3. Verfahren gemäß Anspruch 1, wobei das anorganische Pulver dem Zufuhrabschnitt des Kneters zugegeben wird.

4. Verfahren gemäß Anspruch 1, wobei das anorganische Pulver nicht später als bei 30 % der mittleren Aufenthaltszeit des Inhalts des Kneters zugegeben wird.

5. Verfahren gemäß einem der Ansprüche 1 bis 4, wobei das anorganische Pulver ein Ton ist.

6. Verfahren gemäß Anspruch 5, wobei das anorganische Pulver Kaolin ist.

7. Verfahren gemäß einem der Ansprüche 1 bis 6, wobei der andere Zusatzstoff superabsorbierendes Feinmaterial ist.

## Revendications

1. Procédé pour la production de superabsorbants par polymérisation dans un malaxeur pourvu d'au moins deux arbres parallèles qui comprend également des éléments sur au moins un arbre qui transportent le contenu du malaxeur parallèlement aux arbres d'une section d'alimentation à une section d'évacuation, le procédé comprenant l'ajout d'une poudre inorganique au contenu du malaxeur avant ou pendant la polymérisation et l'ajout d'au moins un autre additif au contenu du malaxeur pendant la polymérisation, la poudre inorganique étant ajoutée au plus tard à la moitié du temps de séjour moyen du contenu du malaxeur et l'autre additif étant ajouté au plus tôt à la moitié de ce temps de séjour.

2. Procédé selon la revendication 1, dans lequel la poudre inorganique est ajoutée à la solution de monomères alimentant le malaxeur.

3. Procédé selon la revendication 1, dans lequel la poudre inorganique est ajoutée à la section d'alimentation du malaxeur.

4. Procédé selon la revendication 1, dans lequel la poudre inorganique est ajoutée au plus tard à 30 % du temps de séjour moyen du contenu du malaxeur.

5. Procédé selon 1"une quelconque des revendications 1 à 4, dans lequel la poudre inorganique est une argile.

6. Procédé selon la revendication 5, dans lequel la poudre inorganique est le kaolin.

7. Procédé selon l'une quelconque des revendications 1 à 6, dans lequel l'autre additif est constitué de fines de superabsorbant.
